# FASCICULE DE BREVET EUROPEEN

(11) **EP 3 353 140 B1**
(45) Date de publication et mention de la délivrance du brevet: **15.03.2023**
(21) Numéro de dépôt: 16775511.5
(22) Date de dépôt: 19.09.2016
(51) Int. Cl.: C07C 17/389, C07C 21/18

(54) **PROCEDE DE PURIFICATION ET DE SECHAGE D'UN FLUX D'HYDROFLUOROOLEFINES**
VERFAHREN ZUR REINIGUNG UND TROCKNUNG EINES HYDROFLUOROLEFINSTROMS
METHOD FOR PURIFYING AND DRYING A HYDROFLUOROOLEFIN STREAM

(30) Priorité: 24.09.2015 FR 1559002
(43) Date de publication de la demande: 01.08.2018
(73) Titulaire: Arkema France, 92700 Colombes (FR)
(72) Inventeur: PIGAMO, Anne, 69340 Francheville (FR); DEUR-BERT, Dominique, 69390 Charly (FR); WENDLINGER, Laurent, 69510 Soucieu en Jarrest (FR)
(74) Mandataire: Hirsch & Partners
(86) Numéro de dépôt international: PCT/EP2016/072142
(87) Numéro de publication internationale: WO 2017/050686

(56) Documents cités:
- EP-A1- 2 796 439
- WO-A1-2015/125877
- WO-A2-2010/001025
- GB-A- 2 439 209
- JP-A- 2012 001 495
- Claudia Arnold: ""Zeolites in VOC Abatement and Industrial Waste Gas Purification"", Zeolites in Chemical Engineering, 1 January 2011 (2011-01-01), pages 1-24, XP055684306, DOI: 10.13140/2.1.1145.1843 Retrieved from the Internet: URL:https://epo.summon.serialssolutions.co m/2.0.0/link/0/eLvHCXMwY2BQSUozMkoBdhx0E83 Bo1XADquFSaqlrmGKQVKSCWhVQDJ4tbuzSYi7eZC7i Q8TgwVsLww0fkHLI5NBs6eguXzQCiXI_SPGwO6AvpG eIeiEW1M90FUlzMA0bWAMPY0RUxpYwsKMQqo23AQZO FyAwgrBqSVCDEypeSIMjkpRqaAFZ6nFCpl5CmH-zgq OScDGHmiETgHYpVdAXKShEJ4IDH8F98RihYDSItB6H nAQKokymLi [retrieved on 2020-04-08]
- Shaoying Qi ET AL: "Predicting humidity effect on adsorption capacity of activated carbon for water-immiscible organic vapors", Advances in Environmental Research, vol. 4, no. 4, 1 November 2000 (2000-11-01), pages 357-362, XP055684321, AMSTERDAM, NL ISSN: 1093-0191, DOI: 10.1016/S1093-0191(00)00033-2
- Uop: "Precautions and Safe Practices for Handling Zeolite Molecular Sieve Adsorbents in Process Units", , 1 January 2003 (2003-01-01), pages 1-16, XP055684330, Retrieved from the Internet: URL:https://offenbar-energy.com/pdf/SIEVE% 20SAFE%20PRACTICES.pdf [retrieved on 2020-04-08]

## Description

### DOMAINE DE L'INVENTION

La présente invention concerne un procédé de purification et de séchage d'un flux d'hydrofluorooléfines à l'aide d'un agent adsorbant. La présente invention concerne également l'utilisation d'un agent adsorbant pour simultanément sécher et purifier un flux d'hydrofluorooléfines.

### ARRIERE-PLAN TECHNIQUE

Le protocole de Montréal pour la protection de la couche d'ozone a conduit à l'abandon de l'utilisation des chlorofluorocarbures (CFC). Des composés moins agressifs pour la couche d'ozone, tels que les hydrofluorocarbures (HFC) ont ainsi remplacé les chlorofluorocarbures. Toutefois ces composés contribuent de manière relativement importante à l'effet de serre. On a donc recherché des composés de substitution efficaces présentant à la fois un faible coefficient ODP (potentiel de déplétion de l'ozone) et un faible coefficient GWP (potentiel de réchauffement climatique). Les hydrofluorooléfines (HFO) ont été identifiées comme des alternatives souhaitables, du fait de leurs faibles valeurs d'ODP et de GWP.

Les hydrofluorocarbones (HFC) et en particulier les hydrofluorooléfines (HFOs), tel que le 2,3,3,3-tétrafluoro-1-propène (HFO-1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. A la différence des CFC et des HCFC, qui sont potentiellement dangereux pour la couche d'ozone, les HFOs ne contiennent pas de chlore et donc ne posent pas de problème pour la couche d'ozone dans le cadre de leurs utilisations.

Il est connu de produire des hydrofluorooléfines ou des hydrofluorocarbures par fluoration d'hydrochlorooléfines ou d'hydrochlorocarbures notamment. Cette fluoration est généralement une fluoration catalytique utilisant l'acide fluorhydrique comme agent fluorant.

Ce type de procédé de production nécessite généralement des étapes de lavage dans des colonnes prévues à cet effet (eau ou solution aqueuse basique) en vue d'éliminer certains produits indésirables (HF résiduel notamment). Ces étapes de lavage peuvent mener à des compositions d'hydrofluorooléfines comprenant de l'eau et/ou de l'humidité.

Par conséquent, les procédés de fabrication de compositions d'hydrofluorooléfines comprennent généralement une ou plusieurs étapes de séchage après les étapes de lavage, de façon à rendre anhydres ou substantiellement anhydres les flux qui seront traités dans les étapes ultérieures. Les méthodes conventionnelles utilisées pour le séchage sont par exemple l'utilisation d'un composé aux propriétés déshydratantes comme le chlorure de calcium, le sulfate de magnésium, le sulfate de sodium, le sulfate de calcium, l'hydroxyde de potassium ou l'oxyde de calcium. D'autres méthodes bien connues impliquent l'utilisation d'un tamis moléculaire, d'un gel de silice ou bien d'un charbon actif.

En outre, les procédés de fabrication de compositions d'hydrofluorooléfines généralement utilisés conduisent à la formation de sous-produits (impuretés) de type composés hydrocarbonés qui nécessite des étapes ultérieures de purification.

Le document JP2013241390 décrit un procédé de purification d'un flux comprenant du 2,3,3,3-tétrafluoro-1-propène (1234yf) obtenu par pyrolyse. Le document WO 2013/115048 décrit un procédé de purification d'un flux de 1-chloro-3,3,3-trifluoropropène (1233zd) à l'aide d'une zéolithe. Dans ce document, on met en oeuvre des étapes séparées de lavage et de séchage avant de pouvoir effectuer l'étape de purification.

Il existe donc un besoin de simplifier le procédé de fabrication de compositions d'hydrofluorooléfines, par exemple en limitant le nombre d'étapes tout en gardant un procédé efficace présentant un rendement satisfaisant, en particulier pour des applications industrielles.

La présente invention vise à proposer un nouveau procédé de purification et de séchage de compositions d'hydrofluorooléfines permettant à la fois de sécher et d'éliminer des impuretés de type hydrocarbures, en particulier hydrocarbures halogénés saturés. Ainsi, dans le procédé selon l'invention, la purification et le séchage sont effectués simultanément en une seule et même étape.<insérer page 2a>

### RESUME DE L'INVENTION

Le document JP 2012/001495 décrit un procédé de purification d'un flux de 2, 3, 3, 3-tétrafluoropropène (1234yf) par mise en contact avec un agent adsorbant, en particulier des tamis moléculaires de type A ayant un diamètre moyen de 5 à 10 Å.

Le document EP 2 796 439 décrit une méthode de purification de (E)-1-chloro-3,3,3-trifluoropropene ((E)-1233zd) à partir d'un flux comprenant des impuretés telles que HCFC-142, 244fa, 245fa, (E)-1234, (Z)-1234 et (Z)-1233zd, en quantité variant de 0,01 % (100 ppm) à 10 %, par mise en contact avec un agent adsorbant, comme les zéolites de type A ou X.

L'invention concerne en premier lieu un procédé de purification et de séchage d'un flux d'hydrofluorooléfine comprenant une hydrofluorooléfine, de l'eau et des impuretés à base de composés carbonés halogénés, caractérisé en ce que ledit flux est mis en contact avec un agent adsorbant choisi parmi des tamis moléculaires qui sont des zéolithes de type X ayant un diamètre de pores allant de 8 Å à 12 Å, et en ce que la purification et le séchage sont effectués en une seule et même étape, ladite hydrofluorooléfine étant le 1-chloro-3,3,3-trifluoropropène (1233zd).

Selon un mode de réalisation, le flux d'hydrofluorooléfine comprenant une hydrofluorooléfine, de l'eau et des impuretés comprend au moins 50% en poids, de préférence au moins 70% en poids, de préférence au moins 90%, de préférence au moins 95% en poids, de préférence au moins 98% en poids, de préférence au moins 99% en poids d'au moins une même hydrofluorooléfine, par rapport au poids total du flux comprenant une hydrofluorooléfine, de l'eau et des impuretés.

Selon un mode de réalisation de l'invention, les impuretés à base de composés carbonés halogénés comprennent au moins un composé choisi parmi le chlorométhane (F40), le fluorométhane (F41), le difluorométhane (F32), le tétrafluorométhane (F14), le trifluorométhane (F23), le 1,1,1-trifluoroéthane (F143a), le 1,1-difluoroéthane (F152a), le pentafluoroéthane (F125), le chloropentafluoroéthane (F115), le 1,1,1,2-tétrafluoroéthane (F134a), le pentafluoropropane (F245), le monochlorotétrafluoropropène (F244), le 1,3,3,3-tétrafluoropropène (F1234ze) isomère E ou Z, le 3,3,3-trifluoropropène (1243zf), le 1,2,3,3,3-pentafluoropropène (F1225ye), la 3,3,3-trifluoropropyne, le 1,3-dichloro-3,3-difluoropropène (F1232zd), le 1,1 -dichloro-3,3-difluoropropène (F1232za), le 1,1-dichloro-1,3,3-trifluoropropane (F243fc), l'isomère cis du 1-chloro-3,3,3-trifluoropropène (Z-1233zd).

Selon un mode de réalisation de l'invention, le flux d'hydrofluorooléfine avant la mise en contact avec l'agent adsorbant comprend de 10 à 10000 ppm massique d'eau et/ou le flux d'hydrofluorooléfine avant la mise en contact avec l'agent adsorbant comprend de 500 à 8000 ppm massique d'impuretés.

Selon un autre mode de réalisation particulier de l'invention, le flux d'hydrofluorooléfine est un flux de 1233zd de configuration E. Selon ce mode de réalisation, les impuretés sont de préférence choisies parmi le pentafluoropropane (F245), le 1,3,3,3-tétrafluoropropène (F1234ze), le 1,3-dichloro-3,3-difluoropropène (F1232zd), le 1,1-dichloro-3,3-difluoropropène (F1232za), le 1,1-dichloro-1,3,3-trifluoropropane (F243fc), seuls ou en mélange, de préférence parmi le pentafluoropropane (F245), le 1,3,3,3-tétrafluoropropène (F1234ze), le 1,3-dichloro-3,3-difluoropropène (F1232zd), le 1,1-dichloro-3,3-difluoropropène (F1232za), le 1,1-dichloro-1,3,3-trifluoropropane (F243fc), le 1-chloro-3,3,3-trifluoropropène de configuration Z (Z-1233zd), seuls ou en mélange.

L'invention concerne également l'utilisation d'un agent adsorbant choisi parmi des tamis moléculaires qui sont des zéolithes de type X ayant un diamètre de pores allant de 8 Å à 12 Å, pour simultanément sécher et purifier un flux d'hydrofluorooléfine comprenant une hydrofluorooléfine, de l'eau et des impuretés à base de composés carbonés halogénés, ladite hydrofluorooléfine étant le 1-chloro-3,3,3-trifluoropropène (1233zd).

Le procédé selon l'invention est simple à mettre en oeuvre, en particulier à l'échelle industrielle.

Le procédé selon l'invention permet d'obtenir une composition d'hydrofluorooléfines de pureté améliorée, par un procédé simplifiée.

Le procédé selon l'invention permet de sécher et purifier un flux d'hydrofluorooléfine en une seule et même étape.

Le procédé selon l'invention est basé sur la découverte surprenante qu'il est possible en une seule et même étape de sécher et de purifier un flux d'hydrofluorooléfine à l'aide d'un agent adsorbant. En effet, jusqu'à présent, le séchage et la purification étaient effectués dans deux étapes distinctes. Il était généralement reconnu par l'homme du métier qu'en présence d'eau ou d'humidité, l'agent adsorbant se gorgeait de cette eau ou de cette humidité et devenait inutilisable pour effectuer une purification.

### DESCRIPTION DE MODES DE REALISATION DE L'INVENTION

L'invention est maintenant décrite plus en détail et de façon non limitative dans la description qui suit.

La présente invention propose un procédé de purification et de séchage d'une hydrofluorooléfine qui est le 1-chloro-3,3,3-trifluoropropène (1233zd), ledit procédé comprenant la mise en contact d'un flux comprenant l'hydrofluorooléfine (espèce d'intérêt), de l'eau et des impuretés à base de composés halogénés (différentes de l'espèce d'intérêt), avec un agent adsorbant choisi parmi des tamis moléculaires qui sont des zéolithes de type X ayant un diamètre de pores allant de 8 Å à 12 Å.

Par « hydrofluorooléfine », au sens de la présente invention, on entend un composé hydrocarboné comprenant au moins une insaturation et au moins un atome de fluor.

L'hydrofluorooléfine comprend 3 atomes de fluor.

Selon un mode de réalisation de l'invention, le flux comprenant une hydrofluorooléfine (espèce d'intérêt), de l'eau et des impuretés avant la mise en contact avec l'agent adsorbant comprend au moins 50% en poids d'au moins une hydrofluorooléfine, de préférence au moins 70% en poids d'au moins une hydrofluorooléfine, de préférence encore au moins 90% en poids d'au moins une hydrofluorooléfine, voire au moins 95% en poids d'au moins une hydrofluorooléfine ou même au moins 98% en poids d'au moins une hydrofluorooléfine ou au moins 99% en poids d'au moins une hydrofluorooléfine, par rapport au poids total du flux comprenant une hydrofluorooléfine, de l'eau et des impuretés.

Par « impureté », il faut entendre une espèce présente, dans le flux comprenant une hydrofluorooléfine, de l'eau et des impuretés, en une quantité minoritaire, de préférence en une quantité inférieure ou égale à 5% en poids, par rapport au poids total dudit flux. La ou les impuretés sont différentes de l'hydrofluorooléfine en tant qu'espèce d'intérêt.

Le flux comprenant une hydrofluorooléfine peut être obtenu par un procédé connu de l'homme du métier, par exemple par un procédé tel que décrit dans le document US 2014/0296585 ou le document WO 2015/104517.

Lors de la fabrication des hydrofluorooléfines, des étapes de lavage sont souvent nécessaires et provoquent l'introduction d'eau ou d'humidité dans le flux d'hydrofluorooléfine ainsi lavé. En outre, des sous-produits (impuretés) sont également obtenus lors de la réaction permettant d'obtenir l'hydrofluorooléfine souhaitée.

Selon un mode de réalisation, le flux comprenant une hydrofluorooléfine, de l'eau et des impuretés à base de composés halogénés comprend moins de 5% en poids d'eau et d'impuretés à base de composés halogénés, de préférence moins de 3% en poids d'eau et d'impuretés à base de composés halogénés, de préférence encore moins de 2% en poids d'eau et d'impuretés à base de composés halogénés, avantageusement moins de 1% en poids d'eau et d'impuretés à base de composés halogénés, par rapport au poids total dudit flux.

Les impuretés sont généralement à base de composés halogénés, saturés ou insaturés, par exemple à base de composés halogénés comprenant un, deux ou trois atomes de carbone.

Parmi les impuretés halogénées contenant un ou deux atomes de carbone, on peut citer le chlorométhane (F40), le fluorométhane (F41), le difluorométhane (F32), le tétrafluorométhane (F14), le trifluorométhane (F23), le pentafluoroéthane (F125), le chloropentafluoroéthane (F115), le 1,1,1,2-tétrafluoroéthane (F134a), le 1,1,1-trifluoroéthane (F143a) et le 1,1-difluoroéthane (F152a).

D'autres impuretés halogénées contenant 3 atomes de carbone peuvent également être formées, telles que le pentafluoropropane (F245), le monochlorotétrafluoropropène (F244), le 1,3,3,3-tétrafluoropropène (F1234ze), le 3,3,3-trifluoropropène (1243zf), le 1,2,3,3,3-pentafluoropropène (F1225ye), la 3,3,3-trifluoropropyne, le 1,3-dichloro-3,3-difluoropropène (F1232zd), le 1,1-dichloro-3,3-difluoropropène (F1232za) et le 1,1-dichloro-1,3,3-trifluoropropane (F243fc).

Lorsque deux isomères existent, un des isomères peut être considéré comme une impureté par rapport à l'autre, en particulier, on peut citer l'isomère cis du 1-chloro-3,3,3-trifluoropropène dans un flux contenant majoritairement du trans-1-chloro-3,3,3-trifluoropropène ou l'isomère cis du 1,3,3,3-tétrafluoropropène dans un flux contenant majoritairement du trans-1,3,3,3-tétrafluoropropène.

Le procédé selon l'invention permet d'obtenir un flux à la fois enrichi en l'hydrofluorooléfine, c'est-à-dire que la proportion molaire d'hydrofluorooléfine (produit d'intérêt) dans le flux initial avant purification et séchage est inférieure à la proportion molaire de ladite hydrofluorooléfine dans le flux après purification, et appauvri en impuretés.

Le flux comprenant une hydrofluorooléfine, de l'eau et des impuretés à base de composés halogénés utilisé dans le procédé selon l'invention peut avant purification comprendre par exemple :
- de 10 à 10000 ppm en poids d'eau, de préférence de 50 à 8000 ppm en poids d'eau, et
- de 500 à 8000 ppm en poids, de préférence de 1000 à 6000 ppm en poids, d'impuretés à base de composés halogénés,
par rapport au poids total du flux d'hydrofluorooléfine.

De préférence, le flux après purification et séchage comprend de 5 à 100 ppm massique d'eau et de 5 à 3000 ppm massique d'impuretés.

L'eau peut par exemple provenir d'une étape précédente de lavage du flux d'hydrofluorooléfines.

Le procédé de purification selon l'invention comprend la mise en contact du flux comprenant l'hydrofluorooléfine, de l'eau et des impuretés avec un agent adsorbant choisi parmi des tamis moléculaires qui sont des zéolithes de type X ayant un diamètre de pores allant de 8 Å à 12 Å.
Les tamis moléculaires, encore appelés zéolithes synthétiques, sont des composés chimiques largement utilisés dans l'industrie comme agents adsorbants, notamment pour sécher des gaz ou des liquides. Ce sont des alumino-silicates métalliques qui possèdent une structure cristalline tridimensionelle constituée par un assemblage de tétraèdres. Ces tétraèdres sont formés par quatre atomes d'oxygène qui occupent les sommets, et qui entourent soit un atome de silicium, soit un atome d'aluminium placé au centre. Ces édifices contiennent généralement des cations pour rendre le système électriquement neutre, tels ceux dérivés du sodium, du potassium ou du calcium.

Les tamis moléculaires qui conviennent sont ceux du type X.

Dans le cas des tamis moléculaires, dits du type A, les tétraèdres sont assemblés de telle manière qu'ils composent un octaèdre tronqué. Ces octaèdres sont eux-mêmes arrangés selon une structure cristalline cubique simple, formant un réseau dont les cavités ont un diamètre approximatif de 11,5 Å. Ces cavités sont accessibles par des ouvertures, ou pores, qui peuvent être partiellement bloquées au moyen de cations. Lorsque ces cations sont dérivés du sodium, ces cavités ont un diamètre d'ouverture de 4 Å, et l'on a alors un tamis moléculaire dit « 4 A ». La structure cristalline d'un tel tamis peut être représentée par la formule chimique suivante:
Na₁₂ [(AlO₂)₁₂(SiO₂)₁₂].XH₂O dans laquelle X qui représente le nombre de molécules d'eau appartenant à la structure (eau de cristallisation) peut atteindre 27, ce qui représente 28,5 % en poids de la zéolithe anhydre.

La taille des ouvertures (ou des pores) peut du reste être modifiée selon les différents types de tamis moléculaire. Ainsi, par échange d'une grande partie des ions sodium d'un tamis moléculaire 4A par des ions potassium, on obtient le tamis moléculaire 3 A, dont les pores ont un diamètre d'environ 3 Å. Le tamis moléculaire 5 A est réalisé en remplaçant les ions sodium par des ions calcium, le diamètre effectif des pores étant alors de l'ordre de 5 Å. La cellule élémentaire de la zéolithe X est un tétraèdre dont les sommets sont occupés par des polyèdres de même type que ceux présents dans la zéolithe A, chacun étant connecté à quatre autres polyèdres grâce à une sous-structure octaédrique, formée par un double-cycle contenant huit atomes d'oxygène. Le centre de chaque arête est toujours occupé par un atome d'oxygène, tandis que les atomes de silicium et d'aluminium occupent les différents sommets des polyèdres. La formule brute est de structure Na₈₈Al₈₈Si₁₀₄O₃₈₄.220H₂O.

Selon l'invention, le flux d'hydrofluorooléfine est un flux de 1-chloro-3,3,3-trifluoropropène (1233zd) et l'agent adsorbant est une zéolithe de type X présentant un diamètres de pores allant de 8 Å à 12 Å.

Selon ce mode de réalisation, l'invention permet d'éliminer en une seule et même étape, l'humidité ou l'eau ainsi que les impuretés organiques, telles que le pentafluoropropane (F245), le 1,3,3,3-tétrafluoropropène (F1234ze), le 1,3-dichloro-3,3-difluoropropène (F1232zd), le 1,1-dichloro-3,3-difluoropropène (F1232za) ou encore le 1,1-dichloro-1,3,3-trifluoropropane (F243fc).

De préférence, le flux de 1-chloro-3,3,3-trifluoropropène est un flux de 1-chloro-3,3,3-trifluoropropène de configuration E (1233zd E). Dans ce cas, le 1-chloro-3,3,3-trifluoropropène de configuration Z (1233zd Z) peut être considéré comme une impureté, complétant la liste des impuretés ci-dessus mentionnée.

Selon ce mode de réalisation, le flux d'hydrofluorooléfine initial (avant procédé de purification et séchage) peut comprendre de 90% à 99% en poids de 1-chloro-3,3,3-trifluoropropène de configuration E (1233zd E), de 0,05 à 3% en poids d'eau, de 1% à 6% en poids de 1-chloro-3,3,3-trifluoropropène de configuration Z (1233zd Z), de 0,1% à 2% en poids de 1,3-dichloro-3,3-difluoropropène (F1232zd), de 0,05% à 2% en poids de pentafluoropropane (F245) et de 0,05% à 2% en poids de 1,1-dichloro-3,3-difluoropropène (F1232za), par rapport au poids total du flux d'hydrofluorooléfine.

Selon un mode de réalisation de l'invention, l'étape de mise en contact est effectuée à une température allant de -20°C à +80°C, de préférence de +10°C à +40°C, et sous une pression de 100 à 2200 kPa, de préférence à pression atmosphérique.

Selon un mode de réalisation, le procédé selon l'invention est mis en oeuvre en phase gazeuse.

La présente invention concerne également l'utilisation d'un agent adsorbant choisi parmi des tamis moléculaires qui sont des zéolithes de type X ayant un diamètre de pores allant de 8 Å à 12 Å au sens défini ci-avant pour simultanément sécher et purifier un flux d'hydrofluorooléfines comprenant une hydrofluorooléfine, de l'eau et des impuretés à base de composés carbonés halogénés, ladite hydrofluorooléfine étant le 1-chloro-3,3,3-trifluoropropène (1233zd).

Les caractéristiques détaillées ci-dessus vis-à-vis du procédé selon l'invention s'appliquent à l'utilisation selon l'invention.

### EXEMPLES

### Exemple 1 : Purification d'un flux de 1234yf (hors de l'invention)

20g de zéolithe NK20^{®} (type A ayant un diamètre de pores de 5 Å) disponible auprès de la Société CECA sont chargées dans un réacteur ayant une longueur de 70 cm et un diamètre intérieur de 1,6 cm ; les 20g de zéolithe recouvrent environ 16cm de la hauteur du réacteur. La zéolithe est préalablement séchée sous un gaz inerte à un débit de 20l/h à 120°C pendant 10 heures.

Le test de purification est ensuite effectué à température ambiante (25°C) et à pression atmosphérique avec un temps de contact d'environ 100 secondes et une vitesse spatiale de 0,3 cm/s.

Plusieurs flux gazeux ont été testés et comparés :
- Flux 1 : 1234yf distillé (pureté de 98,64%) dopé avec 6000 ppm massique de F40.
- Flux 2 : produit brut comprenant 94,42% en poids de 1234yf et du 245cb comprenant en outre 8300 ppm massique de F40.
- Flux 3 : produit brut comprenant 94,55% en poids de 1234yf et du 245cb comprenant en outre 5700 ppm massique de F40, ledit produit brut subissant une étape de barbotage dans de l'eau à 18°C afin d'obtenir un flux gazeux saturé en eau (environ 5000 ppm massique d'eau calculé à partir de la pression de vapeur saturante).
- Flux 4 : produit brut comprenant 94,55% en poids de 1234yf et du 245cb comprenant en outre 4700 ppm massique de F40, ledit produit brut subissant une étape de barbotage dans de l'eau à 25°C afin d'obtenir un flux gazeux saturé en eau (environ 8000 ppm massique d'eau calculé à partir de la pression de vapeur saturante).

Pour chaque test, le temps de contact a été maintenu jusqu'à saturation de la zéolithe. Les impuretés F40 ont été éliminées immédiatement du flux gazeux pour atteindre une teneur inférieure à 20 ppm massique. Une fois la zéolithe saturée, la teneur en F40 du flux augmente à nouveau puisque ledit F40 ne peut plus être éliminé.

Ce type de test permet d'évaluer la capacité d'adsorption de la zéolithe, exprimée en g de F40 par 100g de zéolithe chargée dans le réacteur.

Les résultats sont indiqués dans le tableau 1 ci-dessous.

**Tableau 1 : Capacité d'adsorption du tamis moléculaire NK20^{®}**

| | Flux de 1234yf | | Temps de contact (s) | Durée avant saturation (h) | Capacité d'adsorption (%) |
|---|---|---|---|---|---|
| | F40 | Humidité | | | |
| Flux 1 | 6000 ppm | - | 100 | 40 | 5,1 |
| Flux 2 | 8300 ppm | - | 121 | 37 | 4,4 |
| Flux 3 | 5700 ppm | 5000 ppm | 85 | 30 | 4,0 |
| Flux 4 | 4700 ppm | 8000 ppm | 100 | 29 | 3,3 |

Les résultats du tableau 1 montrent que les flux 3 et 4 comprenant des quantités d'humidité peuvent être purifiés, au même titre que les flux 1 et 2 sans humidité détectable. En effet, la capacité d'absorption est respectivement de 4,0% et de 3,3%, ce qui permet effectivement une purification satisfaisante des impuretés organiques.

### Exemple 2 : purification d'un flux de 1233zd (configuration E)

Des tests ont été réalisés en utilisant 50g de tamis moléculaire et un flux brut de 1233zd E a été mis en contact de la zéolithe pendant 1 heure, à une pression de 0,5 bar et à température ambiante (25°C). Les compositions des flux en entrée et en sortie ont été comparées. Les proportions molaires en chacun des constituants ont été déterminées par une analyse par chromatographie gazeuse, bien connue de l'homme du métier.

Le tableau 2 ci-dessous illustre les résultats obtenus avec une zéolithe G5 (type X ayant un diamètre de pores de 10 Å) disponible auprès de la Société CECA. Un test a également été effectué avec un flux de 1233zd E qui a été préalablement barboté dans de l'eau à 25°C.

**Tableau 2 : purification du 1233zd E**

| Zéolithe | G5 | | G5 (test avec barbotage) | |
|---|---|---|---|---|
| Charge de zéolithe | 81 mL | | 81 mL | |
| 1233zd E introduit | 57,6 g | | 58,4 g | |

| Analyse GC | entrée | sortie | entrée | sortie |
|---|---|---|---|---|
| | %poids | %poids | %poids | %poids |
| F1233zd E | 95,395 | 99,623 | 93,378 | 99,855 |
| F1233zd Z | 3,184 | 0,002 | 4,709 | - |
| F1234ze E | 0,176 | 0,222 | 0,012 | 0,019 |
| F1234ze Z | 0,090 | - | 0,034 | - |
| F245fa | 0,159 | - | 0,084 | - |
| F1232zd E | 0,627 | - | 1,241 | - |
| F243fc | 0,097 | - | 0,182 | - |
| F1232za | 0,134 | - | 0,271 | - |
| autres | 0,138 | 0,036 | 0,234 | 0,038 |

Le tableau 2 ci-dessus montre qu'un flux de 1233zd peut être purifié à l'aide d'un agent adsorbant, tel qu'une zéolithe de type X.

De manière surprenante, les inventeurs ont découvert que le test avec la zéolithe G5 où le flux de 1233zd E comprend de l'eau (ou de l'humidité) permet de purifier le flux et d'obtenir ainsi un flux de 1233zd E présentant une pureté améliorée.

Le tableau 2 montre également que le procédé selon l'invention permet de séparer deux isomères, tels que le F1233zd E et le F1233zd Z.

Le tableau 2 montre également que le procédé selon l'invention permet d'éliminer le F1234ze Z alors que le F1234ze E reste intact et présent dans le flux de F1233zd en sortie.

## Revendications

1. Procédé de purification et de séchage d'un flux d'hydrofluorooléfine comprenant une hydrofluorooléfine, de l'eau et des impuretés à base de composés carbonés halogénés, **caractérisé en ce que** ledit flux est mis en contact avec un agent adsorbant choisi parmi des tamis moléculaires qui sont des zéolithes de type X ayant un diamètre de pores allant de 8 Å à 12 Å, et **en ce que** la purification et le séchage sont effectués en une seule et même étape, et **en ce que** ladite hydrofluorooléfine est le 1-chloro-3,3,3-trifluoropropène (1233zd).

2. Procédé selon la revendication 1, dans lequel le flux d'hydrofluorooléfine comprenant une hydrofluorooléfine, de l'eau et des impuretés comprend au moins 50% en poids, de préférence au moins 70% en poids, de préférence au moins 90%, de préférence au moins 95% en poids, de préférence au moins 98% en poids, de préférence au moins 99% en poids d'au moins une même hydrofluorooléfine, par rapport au poids total du flux comprenant une hydrofluorooléfine, de l'eau et des impuretés.

3. Procédé selon l'une des revendications 1 ou 2, dans lequel les impuretés à base de composés carbonés halogénés comprennent au moins un composé choisi parmi le chlorométhane (F40), le fluorométhane (F41), le difluorométhane (F32), le tétrafluorométhane (F14), le trifluorométhane (F23), le 1,1,1-trifluoroéthane (F143a), le 1,1-difluoroéthane (F152a), le pentafluoroéthane (F125), le chloropentafluoroéthane (F115), le 1,1,1,2-tétrafluoroéthane (F134a), le pentafluoropropane (F245), le monochlorotétrafluoropropène (F244), le 1,3,3,3-tétrafluoropropène (F1234ze) isomère E ou Z, le 3,3,3-trifluoropropène (1243zf), le 1,2,3,3,3-pentafluoropropène (F1225ye), la 3,3,3-trifluoropropyne, le 1,3-dichloro-3,3-difluoropropène (F1232zd), le 1,1-dichloro-3,3-difluoropropène (F1232za), le 1,1-dichloro-1,3,3-trifluoropropane (F243fc), l'isomère cis du 1-chloro-3,3,3-trifluoropropène (Z-1233zd).

4. Procédé selon l'une quelconques des revendications 1 à 3, dans lequel les impuretés sont choisies parmi le pentafluoropropane (F245), le 1,3,3,3-tétrafluoropropène (F1234ze), le 1,3-dichloro-3,3-difluoropropène (F1232zd), le 1,1-dichloro-3,3-difluoropropène (F1232za), le 1,1-dichloro-1,3,3-trifluoropropane (F243fc), seuls ou en mélange, de préférence parmi le pentafluoropropane (F245), le 1,3,3,3-tétrafluoropropène (F1234ze), le 1,3-dichloro-3,3-difluoropropène (F1232zd), le 1,1-dichloro-3,3-difluoropropène (F1232za), le 1,1-dichloro-1,3,3-trifluoropropane (F243fc), le 1-chloro-3,3,3-trifluoropropène de configuration Z (Z-1233zd), seuls ou en mélange, lorsqu'il s'agit d'un flux de (1233zd E).

5. Procédé selon l'une des revendications 1 à 4, dans lequel le flux d'hydrofluorooléfine avant la mise en contact avec l'agent adsorbant comprend de 10 à 10000 ppm massique d'eau.

6. Procédé selon l'une des revendications 1 à 5, dans lequel le flux d'hydrofluorooléfine avant la mise en contact avec l'agent adsorbant comprend de 500 à 8000 ppm massique d'impuretés.

7. Utilisation d'un agent adsorbant choisi parmi des tamis moléculaires qui sont des zéolithes de type X ayant un diamètre de pores allant de 8 Å à 12 Å, pour simultanément sécher et purifier un flux d'hydrofluorooléfine comprenant une hydrofluorooléfine, de l'eau et des impuretés à base de composés carbonés halogénés, ladite hydrofluorooléfine étant le 1-chloro-3,3,3-trifluoropropène (1233zd).

## Patentansprüche

1. Verfahren zur Reinigung und Trocknung eines Hydrofluorolefinstroms, der ein Hydrofluorolefin, Wasser und Verunreinigungen auf der Basis von halogenierten Kohlenstoffverbindungen umfasst, **dadurch gekennzeichnet, dass** der Strom mit einem Adsorptionsmittel in Kontakt gebracht wird, das aus Molekularsieben ausgewählt ist, die Zeolithe vom Typ X mit einem Porendurchmesser von 8 Å bis 12 Å sind, und dadurch, dass die Reinigung und die Trocknung in einem einzigen Schritt durchgeführt werden, und dadurch, dass dieses Hydrofluorolefin 1-Chlor-3,3,3-trifluoropropen (1233zd) ist.

2. Verfahren nach Anspruch 1, wobei der Hydrofluorolefinstrom, der ein Hydrofluorolefin, Wasser und Verunreinigungen umfasst, mindestens 50 Gew.-%, vorzugsweise mindestens 70 Gew.-%, vorzugsweise mindestens 90 Gew.-%, vorzugsweise mindestens 95 Gew.-%, vorzugsweise mindestens 98 Gew.-%, vorzugsweise mindestens 99 Gew.-% mindestens eines gleichen Hydrofluorolefins umfasst, bezogen auf das Gesamtgewicht des Stroms, der ein Hydrofluorolefin, Wasser und Verunreinigungen umfasst.

3. Verfahren nach einem der Ansprüche 1 oder 2, wobei die Verunreinigungen auf der Basis von halogenierten Kohlenstoffverbindungen mindestens eine Verbindung umfassen, die ausgewählt ist aus Chlormethan (F40), Fluormethan (F41), Difluormethan (F32), Tetrafluormethan (F14), Trifluormethan (F23), 1,1,1-Trifluorethan (F143a), 1,1-Difluorethan (F152a), Pentafluorethan (F125), Chlorpentafluorethan (F115), 1,1,1,2-Tetrafluorethan (F134a), Pentafluorpropan (F245), Monochlortetrafluorpropen (F244), 1,3,3,3-Tetrafluorpropen (F1234ze) E- oder Z-Isomer, 3,3,3-Trifluorpropen (1243zf) 1,2,3,3,3-Pentafluorpropen (F1225ye, la 3,3, 3-Trifluorpropin, 1,3-Dichlor-3,3-difluorpropen (F1232zd), 1,1-Dichlor-3,3-difluorpropen (F1232za), 1,1-Dichlor-1,3,3-trifluorpropan (F243fc), dem cis-Isomer von 1-Chlor-3,3,3-trifluorpropen (Z-1233zd).

4. Verfahren nach einem der Ansprüche 1 bis 3, wobei die Verunreinigungen ausgewählt sind aus Pentafluorpropan (F245), 1,3,3,3-Tetrafluorpropen (F1234ze), 1,3-Dichlor-3,3-difluorpropen (F1232zd), 1,1-Dichlor-3,3-difluorpropen (F1232za), 1,1-Dichlor-1,3,3-trifluorpropan (F243fc), einzeln oder im Gemisch, vorzugsweise aus Pentafluorpropan (F245), 1,3,3,3-Tetrafluorpropen (F1234ze), 1,3-Dichlor-3,3-difluorpropen (F1232zd), 1,1-Dichlor-3,3-difluorpropen (F1232za), 1,1-Dichlor-1,3,3-trifluorpropan (F243fc), 1-Chlor-3,3,3-trifluorpropen der Z-Konfiguration (Z-1233zd), einzeln oder im Gemisch, wenn es sich um einen Strom von (1233zd E) handelt.

5. Verfahren nach einem der Ansprüche 1 bis 4, wobei der Hydrofluorolefinstrom vor dem Kontakt mit dem Adsorptionsmittel 10 bis 10.000 ppm Massenanteil Wasser umfasst.

6. Verfahren nach einem der Ansprüche 1 bis 5, wobei der Hydrofluorolefinstrom vor dem Kontakt mit dem Adsorptionsmittel 500 bis 8.000 ppm Massenanteil Verunreinigungen umfasst.

7. Verwendung eines Adsorptionsmittels, ausgewählt aus Molekularsieben, die Zeolithe vom X-Typ mit einem Porendurchmesser von 8 Å bis 12 Å sind, zur gleichzeitigen Trocknung und Reinigung eines Hydrofluorolefinstroms, der ein Hydrofluorolefin, Wasser und Verunreinigungen auf der Basis von halogenierten Kohlenstoffverbindungen umfasst, wobei es sich bei dem Hydrofluorolefin um 1-Chlor-3,3,3-trifluoropropen (1233zd) handelt.

## Claims

1. A method for purifying and drying a hydrofluoroolefin stream comprising a hydrofluoroolefin, water and impurities based on halogenated carbon compounds, **characterized in that** said stream is placed in contact with an adsorbent selected from molecular sieves which are zeolites of type X having a pore diameter ranging from 8 Å to 12 Å and **in that** purification and drying are performed in one same step, and **in that** said hydrofluoroolefin is 1-chloro-3,3,3-trifluoropropene (Z-1233zd).

2. The method according to claim 1, wherein the hydrofluoroolefin stream comprising a hydrofluoroolefin, water and impurities comprises at least 50% by weight, preferably at least 70% by weight, preferably at least 90%, preferably at least 95% by weight, preferably at least 98% by weight, preferably at least 99% by weight of at least one same hydrofluoroolefin, relative to the total weight of the stream comprising a hydrofluoroolefin, water and impurities.

3. The method according to one of claims 1 or 2, wherein the impurities based on halogenated carbon compounds comprise at least one compound selected from chloromethane (F40), fluoromethane (F41), difluoromethane (F32), tetrafluoromethane (F14), trifluoromethane (F23), 1,1,1-trifluoroethane (F143a), 1,1-difluoroethane (F152a), pentafluoroethane (F125), chloropentafluoroethane (F115), 1,1,1,2-tetrafluoroethane (F134a), pentafluoropropane (F245), monochlorotetrafluoropropene (F244), 1,3,3,3-tetrafluoropropene (F1234ze) isomer E or Z, 3,3,3-trifluoropropene (1243zf), 1,2,3,3,3-pentafluoropropene (F1225ye), 3,3,3-trifluoropropyne, 1,3-dichloro-3,3-difluoropropene (F1232zd), 1,1-dichloro-3,3-difluoropropene (F1232za), 1,1-dichloro-1,3,3-trifluoropropane (F243fc), cis isomer of 1-chloro-3,3,3-trifluoropropene (Z-1233zd).

4. The method according to any one of claims 1 to 3, wherein the impurities are selected from pentafluoropropane (F245), 1,3,3,3-tetrafluoropropene (F1234ze), 1,3-dichloro-3,3-difluoropropene (F1232zd), 1,1-dichloro-3,3-difluoropropene (F1232za), 1,1-dichloro-1,3,3-trifluoropropane (F243fc), alone or in a mixture, preferably from pentafluoropropane (F245), 1,3,3,3-tetrafluoropropene (F1234ze), 1,3-dichloro-3,3-difluoropropene (F1232zd), 1,1-dichloro-3,3-difluoropropene (F1232za), 1,1-dichloro-1,3,3-trifluoropropane (F243fc), 1-chloro-3,3,3-trifluoropropene of Z configuration (Z-1233zd), alone or in a mixture when it is a stream of (1233zd E).

5. The method according to one of claims 1 to 4, wherein the hydrofluoroolefin stream, before being placed in contact with the adsorbent, comprises from 10 to 10,000 ppm by mass of water.

6. The method according to one of claims 1 to 5, wherein the hydrofluoroolefin stream before being placed in contact with the adsorbent comprises from 500 to 8,000 ppm by mass of impurities.

7. Use of an adsorbent selected from molecular sieves which are zeolites of type X having a pore diameter ranging from 8 Å to 12 Å for the simultaneous drying and purification of a hydrofluoroolefin stream comprising a hydrofluoroolefin, water and impurities based on halogenated carbon compounds, said hydrofluoroolefin being 1-chloro-3,3,3-trifluoropropene (Z-1233zd).
